# EUROPEAN PATENT APPLICATION

(11) **EP 4 672 262 A1**
(43) Date of publication of application: **31.12.2025**
(21) Application number: 24760675.9
(22) Date of filing: 20.02.2024
(51) Int. Cl.: G16H 50/20, G16H 10/60, G10L 15/26, G06V 30/24, G06F 40/30, G06N 3/08, G06N 20/00, G10L 13/08

(54) **MEDICAL PROMPT GENERATION DEVICE AND METHOD**

(30) Priority: 20.02.2023 KR 20230022426; 19.02.2024 KR 20240023701
(71) Applicant: Seoul National University Hospital, Seoul 03080 (KR)
(72) Inventor: KIM, Joonghee, Seongnam-si, Gyeonggi-do 13620 (KR)
(74) Representative: V.O.
(86) International application number: PCT/KR2024/095427
(87) International publication number: WO 2024/177487

(57) **Abstract**

A medical prompt generation device and method according to one embodiment are disclosed. The medical prompt generation device according to one embodiment comprises: one or more processors; and one or more memories for storing instructions executed by the one or more processors, wherein the one or more processors generate a first sub-prompt on the basis of values output by means of an artificial intelligence model.

## Description

### Technical Field

The disclosed embodiments relate to a medical prompt generation device and method, and more particularly, to a technique for generating improved prompts for facilitating analysis of medical analysis results output based on an artificial intelligence model.

### Cross-Reference to Related Application

This application claims priority to Korean Provisional Patent Application No. 10-2023-0022426 filed on February 20, 2023, and Korean Patent Application No.10-2024-0023701 filed on February 19, 2024, the disclosures of which are incorporated by reference herein in their entirety.

### Background Art

In recent years, health care technology has advanced in connection with medical artificial intelligence. In particular, artificial intelligence outputs a plurality of values for evaluating a health condition of a specific individual in various aspects, which facilitates detailed and accurate evaluation of a patient's condition.

However, these value-based analysis results can be poorly interpreted and utilized. This is because, first, one or more values are difficult for a user to understand without expertise. Second, it is not easy to consider contextual information when interpreting the findings output by the artificial intelligence model even though the findings should be interpreted in a clinical context. Finally, it is also not easy to provide these interpretations in a way that suits the user's specific purpose. For example, certain users may want to know what treatment they should take right now rather than assessing the patient's condition.

In other words, in order to maximize the effectiveness of medical artificial intelligence, the results derived by the artificial intelligence model must be meaningfully delivered to the user. However, the current research only focuses on improving the accuracy of the model and does not consider the user aspect.

With the recent development of large-scale language models, it is possible to receive useful answers when transmitting appropriate information and instructions (prompts) to the language model. In the medical field, the language model can be usefully used for clinical treatment, but it is important to provide appropriate prompts. However, a method for providing optimal prompts in a situation where medical artificial intelligence is used together has not been proposed.

### Detailed Description of the Invention

### Technical Solution

Disclosed embodiments are techniques for generating a medical prompt. Specifically, disclosed embodiments are techniques for creating a medical prompt for effective clinical use of the numerical outputs from an artificial intelligence model that performs medical analysis.

### Summary of the Invention

According to an embodiment, there is provided a medical prompt generation device, comprising one or more processors, and one or more memories for storing instructions executed by the one or more processors, wherein the one or more processors generate a first sub-prompt based on values output by means of an artificial intelligence model.

The one or more processors may generate a medical prompt by inputting the first sub-prompt into a language model.

The one or more processors may generate a second sub-prompt based on user-provided information, generate a third sub-prompt based on a preset setting environment for setting a style of the medical prompt, and generate the medical prompt based on the first sub-prompt and at least parts of the second sub-prompt and the third sub-prompt.

The one or more processors may generate the first sub-prompt based on values in a vector format including, as an element, at least one of a type and a predicted probability of a disease expressed in a numerical form.

The one or more processors may generate the second sub-prompt based on the user-provided information including at least one of an age, a gender, a symptom, a medical history, an underlying disease, a social history, a differential diagnosis request, an emergency treatment request, a history taking request, a physical examination request, a medication request, and a treatment request.

The one or more processors may generate the third sub-prompt based on the setting environment including at least one of a persona of the medical prompt, the context of the persona, and the setting of the persona.

The values may be output by inputting the user-provided information into the artificial intelligence model.

The one or more processors may be configured to generate the first sub-prompt by segmenting the values into categorical values through single-value segmentation or multi-value segmentation, and perform segmental interpretation.

The one or more processors may generate the first sub-prompt when a vector corresponding to the values or an element of the vector satisfies a predefined condition function.

The one or more processors may generate the medical prompt of a sentence by inputting the first sub-prompt and at least parts of the second sub-prompt and the third sub-prompt into a language model.

A method for generating a medical prompt according to an embodiment is a method performed by the medical prompt generation device including one or more processors and one or more memories for storing instructions executed by the one or more processors, the method including generating a first sub-prompt based on values output by an artificial intelligence model.

The method may further include inputting the first sub-prompt into a language model to generate the medical prompt.

The method may further include generating a second sub-prompt based on user-provided information, and generating a third sub-prompt based on a preset setting environment for setting a style of the medical prompt, wherein the generating of the medical prompt may include generating the medical prompt based on the first sub-prompt and at least parts of the second sub-prompt and the third sub-prompt.

The generating of the first sub-prompt may include generating the first sub-prompt based on values in a vector format including, as an element, at least one of a type and a predicted probability of a disease expressed in a numerical form.

The generating of the second sub-prompt may include generating the second sub-prompt based on the user-provided information including at least one of an age, a gender, a symptom, a medical history, an underlying disease, a social history, a differential diagnosis request, an emergency treatment request, a history taking request, a physical examination request, a medication request, and a treatment request.

The generating of the third sub-prompt may include generating the third sub-prompt based on a setting environment including at least one of a persona of the medical prompt, the context of the persona, and the setting of the persona.

The values may be output by inputting the user-provided information into the artificial intelligence model.

The generating of the first sub-prompt may include generating the first sub-prompt by segmenting the values into categorical values through single-value segmentation or multi-value segmentation, and performing segmental interpretation.

The generating of the first sub-prompt may include generating the first sub-prompt when the vector corresponding to the values or the element of the vector satisfies a predefined condition function.

The generating of the medical prompt may include generating the medical prompt of a sentence by inputting the first sub-prompt and at least parts of the second sub-prompt and the third sub-prompt to a language model.

### Effects of the Invention

In one aspect, the disclosed embodiments may improve the prompt including the medical diagnosis result to help the user easily understand the medical diagnosis result.

In another aspect, the disclosed embodiments may emphasize the significance of value sections by incorporating the segmental interpretation to the prompt including the medical diagnosis result.

In another aspect, the disclosed embodiments may set the persona of the prompt to provide the medical diagnostic result tailored to the user and the user environment.

In another aspect, the disclosed embodiments may provide an optimal prompt while using the medical artificial intelligence together.

### Brief Description of the Drawings

FIG. 1 is a block diagram of a medical prompt generation device according to an embodiment.
FIG. 2 is a block diagram of a software unit of a processor according to an example.
FIG. 3 is a block diagram of a software unit of a processor according to an additional example.
FIG. 4 is a flowchart of a method of generating a medical prompt according to an embodiment.
FIG. 5 is a flowchart of a method of generating a medical prompt according to an additional embodiment.

### Embodiments of the Invention

The following description is provided to facilitate a comprehensive understanding of a method, a device, and/or a system described herein. However, this is merely an example, and the present invention is not limited thereto.

In describing the embodiments, detailed descriptions of well-known technologies related to the present invention will be omitted when it is determined that the detailed descriptions may unnecessarily obscure the subject matter of the embodiments.

The terms described below are defined in consideration of functions in the present invention, which may vary depending on the intention or practice of a user, an operator, or the like. Therefore, the definition should be based on the content throughout the specification. The terms used herein are only for describing the embodiments and are not for limiting the same.

Unless explicitly used otherwise, the singular forms include plural forms. It should be understood that the first, second, and the like are used only for distinguishing various elements and are not limited by the terms.

The device of the present invention may be entirely hardware, or may be partially hardware and partially software. For example, a unit may collectively refer to a device for transmitting and receiving data of a specific format and content through electronic communication and software related thereto. In this specification, the terms "unit", "module", "server", "system", "device", and "terminal" are intended to refer to a combination of hardware and software driven by the hardware. For example, the hardware herein may be a data processing device including a central processing unit (CPU) or another processor. In addition, the software driven by hardware may refer to a running process, object, executable, thread of execution, program, or the like.

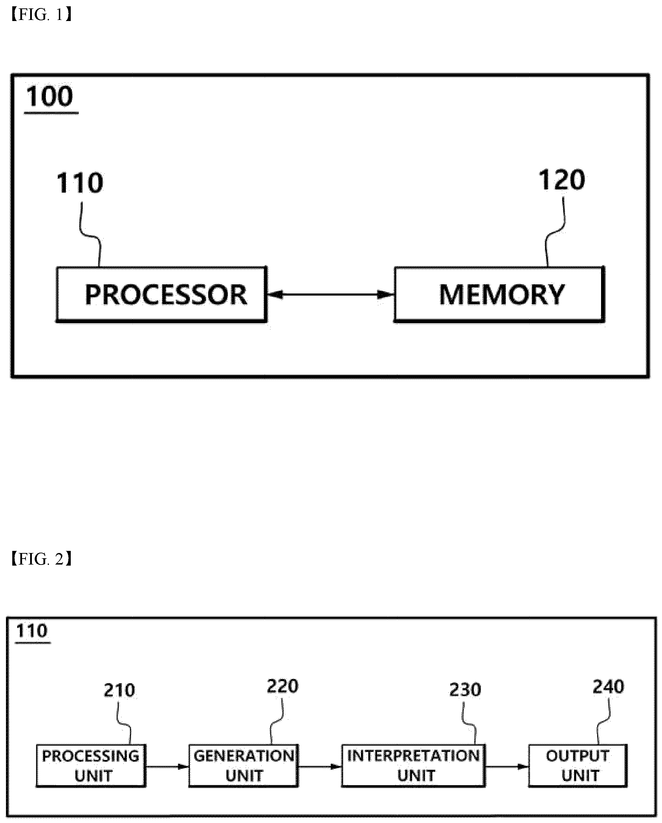

FIG. 1 is a block diagram of a medical prompt generation device 100 according to an embodiment.

Referring to FIG. 1, the medical prompt generation device 100 includes a processor 110 and a memory 120.

The processor 110 generates a first sub-prompt based on values output by an artificial intelligence model.

The processor 110 may generate the first sub-prompt based on a vector including, as an element, the values indicating a type and/or a predicted probability of a disease.

As an example, the processor 110 may generate the first sub-prompt by applying a corresponding transformation to the values output by the artificial intelligence model. The processor 110 may generate the first sub-prompt by mapping the type and the predicted probability of a disease output by the artificial intelligence model to a specific expression in a natural language format.

As a specific example, when the value output by the artificial intelligence model is derived as 70, the processor 110 may generate the first sub-prompt of "the risk of myocardial infarction (disease type) is 70% (predicted probability)."

In this case, the value may be a result in a numerical format which is output when information (e.g., user-provided information to be described below) acquired by the processor 110 is input to the artificial intelligence model.

The valued output by the artificial intelligence model may be expressed in various forms, such as numerical data as diagnostic results, logical values as categorical data, and classes corresponding to sections.

The values for the sub-prompt have been described as being generated based on the output result from the artificial intelligence model, but this is an example. The values may be generated based on the medical result directly input by a user. Thus, the values are not limited to the above.

In addition, the type and number of artificial intelligence models is not limited. The artificial intelligence model may be a model including at least one of a simple conditional expression, a regression model, a machine learning model, and a neural network model.

In another example, the processor 110 may generate the first sub-prompt by applying a segmented transformation to values in a vector format.

The processor 110 may generate the first sub-prompt by segmenting the values into categorical values through single-value segmentation or multi-value segmentation and performing segmental interpretation.

For example, the processor 110 may generate the first sub-prompt of "the risk of myocardial infarction (disease type) is very low (segmental analysis)" in response to the values in a vector format of the above example.

That is, the processor 110 may generate the first sub-prompt that allows the user to clinically interpret the meaning of the segment through segmental interpretation of the value result.

The processor 110 may omit the generation of the first sub-prompt in response to the categorical segmentation of numerical results corresponding to the segments that are clinically insignificant or have low confidence.

The processor 110 may generate the first sub-prompt when the vector having the value as an element or an element of the vector satisfies a predefined condition function. In other words, the processor 110 may initiate generation of the first sub-prompt when the vector or the element of the vector satisfies the predefined condition function.

Although the processor 110 determines whether the condition function is satisfied by the vector, the processor 110 may start the generation of the first sub-prompt depending on whether the data having an additional component other than the vector satisfies the condition function.

In this case, the condition function may vary depending on the purpose of use of the medical prompt and may include a simple conditional expression, machine learning, and a deep learning-based artificial intelligence model.

The additional component may further include user-provided information, a setting environment, and the like, which will be described below.

The processor 110 may generate the medical prompt by inputting the first sub-prompt into the language model.

The processor 110 may generate the medical prompt by inputting the first sub-prompt and a request for the first sub-prompt into the language model.

That is, the processor 110 may generate a more sophisticated medical prompt than the previous first sub-prompt by using the language model.

For example, the processor 110 may generate the medical prompt by inputting, into the language model, an instruction for additionally requesting at least one of summary and editing of the first sub-prompt, first aid, and necessary additional tests.

The processor 110 may generate the second sub-prompt based on the obtained user-provided information.

The user-provided information may include at least one of an age, a gender, a symptom, a medical history, an underlying disease, a social history, a differential diagnosis request, an emergency treatment request, a history taking request, a physical examination request, a medication request, and a treatment request.

The user-provided information, which is information provided in real time by a user input, may be generated based on user-provided information expressed in a natural language form input from a terminal of the user.

The user-provided information may be generated by converting, into text, not only the user input in a text format, but also a user voice, an uploaded image, and the like.

The processor 110 may extract only preset items from the user-provided information to generate the second sub-prompt.

The processor 110 may generate the second sub-prompt by converting part or all of the user-provided information into structured data.

For example, if the user-provided information is "a 45-year-old male patient came to the emergency room in an ambulance with a chest pain that occurred two days ago," the corresponding structured data may be "age: 45; gender: male; main symptom: chest pain; occurrence time: 48 hours ago."

The processor 110 may refine the user-provided information to generate the second sub-prompt.

The processor 110 may generate the second sub-prompt by refining the user-provided information of the above example to "a 45-year-old man visited the hospital with a chest pain that occurred 48 hours ago."

That is, the processor 110 may generate the second sub-prompt by replacing the input user-provided information with the refined words in a predefined word list.

Although the processor 110 generates the second sub-prompt by extracting only some items from the user-provided information, the processor 110 may also generate the second sub-prompt based on some or all of the items.

The processor 110 may generate the third sub-prompt based on a preset setting environment for setting a style of the medical prompt.

The setting environment may include at least one of the persona of the medical prompt, the context of the persona, and the setting of the persona. That is, the setting environment may be a predetermined option according to a user's preference.

In a specific example, the persona may be set based on a character of any one of a doctor, a caregiver, a patient, and a paramedic.

The context of the persona relates to a use environment, and may be set to at least one of a first aid site, an outpatient clinic, a hospital room, an emergency room, and an intensive care unit.

The setting of the persona may include at least one of a basic request type (e.g., differential diagnosis, first aid, additional history taking, additional physical examination, additional test, medication, and treatment), an answer style (e.g, language, medical terminology usage, level of detail in the description), and a medical instruction.

Specifically, the processor 110 may generate the third sub-prompt showing a request related to an item included in the setting environment in natural language.

As a specific example, if the setting environment is set to " (4, 1)", the processor 110 may generate the third sub-prompt, such as "Please let me know briefly (detail level) from the paramedic's perspective (persona)."

The processor 110 may further use the first sub-prompt, as well as at least parts of the second and third sub-prompts to generate the medical prompt.

That is, the processor 110 may generate the medical prompt by listing the first sub-prompt and at least parts of the second sub-prompt and the third sub-prompt.

For example, based on the three sentences respectively corresponding to the first to third sub-prompts of "The electrocardiogram shows a very high risk of myocardial infarction", "Please tell me about the first aid for the 45-year-old male patient who reported chest pain", and "Please tell us briefly from the paramedic's perspective", the processor 110 may generate the medical prompt, such as "The electrocardiogram shows a very high risk of myocardial infarction. Please tell us about the first aid for the 45-year-old male patient who reported chest pain. Please tell us briefly from the paramedic's perspective".

The processor 110 may generate the medical prompt by combining the first sub-prompt with at least parts of the second sub-prompt and the third sub-prompt.

The processor 110 may generate the medical prompt of a sentence by inputting at least parts of the second sub-prompt and the third sub-prompt and the first sub-prompt to the language model.

The processor 110 may generate the medical prompt based on the first to third sub-prompts of the foregoing example, such as "Please briefly tell us about the first aid that paramedics will consider when a 45-year-old male patient who reported chest pain has a very high risk of myocardial infarction."

The processor 110 may input the first to third sub-prompts to the language model in a stepwise manner and additionally request editing. For example, the processor 110 may input the first to third sub-prompts to the language model and may request a summarized sub-prompt as the medical prompt.

The language model, which is a huge model, may be pre-conditioned through fine tuning and prompt engineering, to provide the user with expert-level interpretation when processing the medical prompt.

The processor 110 may input the summarized sub-prompt into the language model to generate an additional medical prompt that requires additional instructions for the summarized sub-prompt.

For example, the processor 110 may generate an additional medical prompt requesting guidance and/or explanation of the patient's differential diagnosis, additional test, and first aid based on the medical prompt described above.

The additional medical prompt is preferably generated by a language model different from the language model that generated the previously generated medical prompt.

The memory 120 stores one or more instructions executed by the processor 110.

The memory 120 may store various data used by the processor 110. For example, the memory 120 may include software (e.g., input data or output data for a program executed by the processor 110 and/or instructions related to the program).

The memory 120 may include volatile memory or nonvolatile memory.

FIG. 2 is a block diagram of a software unit of the processor 110 according to an example.

Referring to FIG. 2, the processor 110 may include a processing unit 210, a generation unit 220, an interpretation unit 230, and an output unit 240.

Unlike the illustrated example, the processing unit 210, the generation unit 220, the interpretation unit 230, and the output unit 240 in FIG. 2 may not be clearly distinguished in specific operations.

The processing unit 210 may generate the first sub-prompt based on the value output by the artificial intelligence model.

In an example, when the processing unit 210 determines that the value output by the artificial intelligence model has clinical significance, the processing unit 210 may generate the first sub-prompt by applying the corresponding transformation to the value.

When the value output by the artificial intelligence model is an estimated value for a measurement index, the processing unit 210 may determine that the value has clinical significance and apply the corresponding transformation to the value.

As a specific example, the processing unit 210 may generate the first sub-prompt including the value output by the artificial intelligence model for predicting the left ventricular ejection fraction.

In another example, when the processing unit 210 determines that the segment where the value output by the artificial intelligence model belongs has clinical significance, the processing unit 210 may generate the first sub-prompt by applying the segmented transformation to the value.

When the value output by the artificial intelligence model is calculated as a score based on a predefined evaluation scale, the processing unit 210 may apply the segmented transformation to the value.

As a specific example, the processing unit 210 may categorize the value output by the artificial intelligence model for predicting the cardiac function risk, and generate the first sub-prompt based on the reinterpreted value.

The generation unit 220 may generate the medical prompt based on the first sub-prompt.

The generation unit 220 may generate the medical prompt including an instruction for requesting information on a specific topic or a task to be performed by the language model for the first sub-prompt.

The language model may include a generative artificial intelligence model.

The interpretation unit 230 may input the medical prompt into the language model to provide a corresponding output sentence.

In this case, the language model used by the interpretation unit 230 may be a model learned to interpret clinical findings with respect to the value output by the artificial intelligence model. For example, the language model of the interpretation unit 230 may be a model learned to interpret the value output by the artificial intelligence model through medical expertise.

The language model used by the interpretation unit 230 may be a model pre-learned on the environment and preference of the user analyzed to have the value output by the artificial intelligence model. For example, the language model of the interpretation unit 230 is a model learned on pre-input information of the user's lifestyle (e.g., smoking, drinking).

That is, the language model may be pre-conditioned through fine tuning, prompt engineering to allow the user to be provided with expert-level interpretation reflecting the personal environment when processing the medical prompt.

The language model of the interpretation unit 230 may be the same as or different from the language model used by the generation unit 220.

The output unit 240 may provide an output of the interpretation unit 230. The output unit 240 may be connected to a terminal of the user who drives the artificial intelligence model and may display the output of the interpretation unit 230 on the screen of the terminal. The output unit 230 may provide the output of the interpret unit 230 as a means for transmitting information to the user regardless of a format such as text or voice.

The output unit 240 may transmit the values of the artificial intelligence model and/or the interpretation of the values to the user in conjunction with the external electronic medical record.

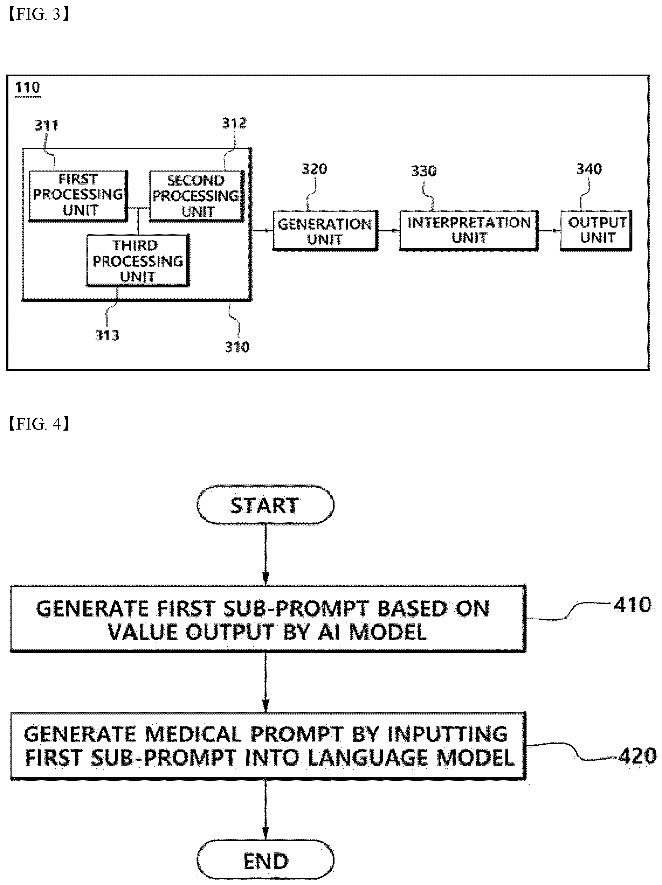

FIG. 3 is a block diagram of a software unit of the processor 110 according to a further example.

Referring to FIG. 3, the processor 110 may include a processing unit 310, a generation unit 320, an interpretation unit 330, and an output unit 340. The processing unit 310 may include a first processing unit 311, a second processing unit 312, and a third processing unit 313.

The interpretation unit 330 and the output unit 340 of FIG. 3 are components that perform the same functions as the interpretation unit 230 and the output unit 240 of FIG. 2, and redundant description thereof will be omitted.

The first processing unit 311 may generate a first sub-prompt based on the value output by the artificial intelligence model. The first processing unit 211 may generate the first sub-prompt representing the clinical findings without distortion of the value output from the artificial intelligence.

The second processing unit 312 may generate the second sub-prompt based on the user-provided information. The second processing unit 212 may generate the second sub-prompt that provides complementary information about the user other than the analytical value through the user-provided information.

The third processing unit 313 may generate the third sub-prompt based on the preset setting environment. The third processing unit 213 may generate the third sub-prompt that provides the environment and preference information of the user.

The preset setting environment may refer to a set of options for adjusting the style of the medical prompt according to user definition.

The value used in the first processing unit 311 is preferably a value result for the user who is the same as the user of the second processing unit 312 and the third processing unit 313.

The generation unit 320 may generate the medical prompt based on the sub-prompt generated by at least one of the first processing unit 311 to the third processing unit 313.

That is, the generation unit 320 may generate the medical prompt based on at least one of the first sub-prompt to the third sub-prompt.

Preferably, the generation unit 320 may generate the medical prompt from a set of sub-prompts that necessarily include the first sub-prompt, but optionally include the second sub-prompt and/or the third sub-prompt.

For example, the generation unit 320 may generate the medical prompt based on the first sub-prompt and the second sub-prompt. For another example, the generation unit 320 may generate the medical prompt based on the second sub-prompt and the third sub-prompt. For another example, the generation unit 320 may generate the medical prompt based on the first, second, and third sub-prompts.

The generation unit 320 may list the obtained sub-prompts to generate the medical prompt. In this case, the order of listing may vary.

The generation unit 320 may input the obtained sub-prompts into the language model to generate the medical prompt of a sentence. In this case, the order of those in the sentence may vary.

FIG. 4 is a flowchart of a method of generating a medical prompt according to an embodiment.

Referring to FIG. 4, the method of FIG. 4 may be performed by the processor 110 of FIG. 2.

First, the processor 110 generates the first sub-prompt based on the value output by the artificial intelligence model (410).

Then, the processor 110 inputs the first sub-prompt into the language model to generate the medical prompt (420).

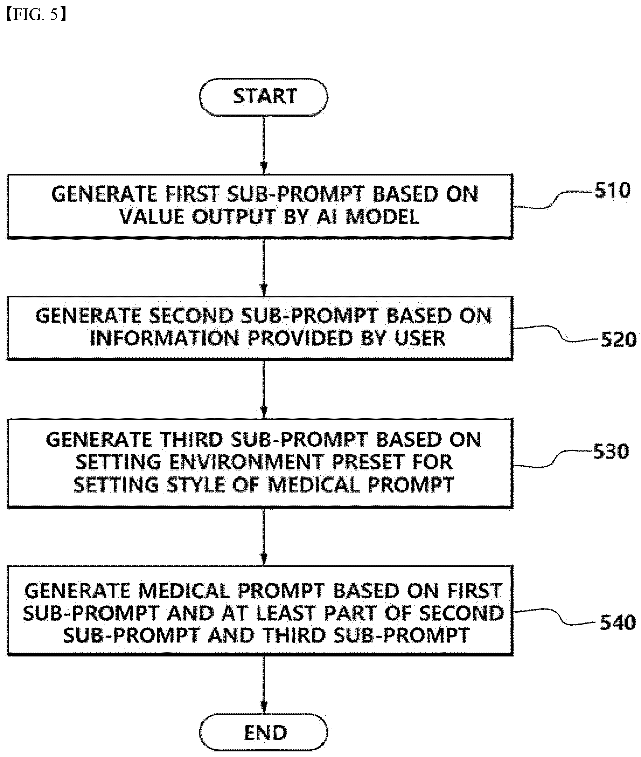

FIG. 5 is a flowchart of a method of generating a medical prompt according to an embodiment.

Referring to FIG. 5, the method of FIG. 5 may be performed by the processor 110 of FIG. 3.

First, the processor 110 generates the first sub-prompt based on the value output by the artificial intelligence model (510).

The processor 110 then generates the second sub-prompt based on the obtained user-provided information (520).

The processor 110 then generates the third sub-prompt based on the preset setting environment for setting the style of the medical prompt (530).

The processor 110 then generates the medical prompt based on the first sub-prompt and at least parts of the second sub-prompt and the third sub-prompt (540).

Although the method is divided into a plurality of steps in FIG. 4 and FIG. 5, at least some of the steps may be performed in different orders, may be combined with other steps and performed together, may be omitted, may be divided into detailed steps, or may be performed by adding one or more steps that are not illustrated herein.

While the exemplary embodiments of the present invention have been described in detail above, those skilled in the art will understand that various modifications can be made to the above-described embodiments without departing from the scope of the present invention. Therefore, the scope of the present invention should not be limited to the described embodiments, but should be defined by the following claims and their equivalents.

### Industrial Applicability

The medical prompt generation device and method according to an embodiment are applicable to the digital medical industry since it is possible to generate a medical prompt for easily interpreting values for a medical result output by an artificial intelligence model.

## Claims

1. A medical prompt generation device comprising:
one or more processors; and
one or more memories for storing instructions executed by the one or more processors, wherein the one or more processors generate a first sub-prompt based on values output by an artificial intelligence model.

2. The medical prompt generation device of claim 1, wherein the one or more processors generate a medical prompt by inputting the first sub-prompt into a language model.

3. The medical prompt generation device of claim 1, wherein the one or more processors: generate a second sub-prompt based on user-provided information; generate a third sub-prompt based on a preset setting environment for setting a style of the medical prompt; and
generate the medical prompt based on the first sub-prompt and at least parts of the second sub-prompt and the third sub-prompt.

4. The medical prompt generation device of claim 1, wherein the one or more processors generate the first sub-prompt based on values in a vector format including, as an element, at least one of a type and a predicted probability of a disease expressed in a numerical form.

5. The medical prompt generation device of claim 3, wherein the one or more processors generate the second sub-prompt based on user-provided information including at least one of an age, a gender, a symptom, a medical history, an underlying disease, a social history, a differential diagnosis request, an emergency treatment request, a history taking request, a physical examination request, a medication request, and a treatment request.

6. The medical prompt generation device of claim 3, wherein the one or more processors generate the third sub-prompt based on a setting environment including at least one of a persona of the medical prompt, a context of the persona, and a setting of the persona.

7. The medical prompt generation device of claim 3, wherein the values are output when the user-provided information is input to the artificial intelligence model.

8. The medical prompt generation device of claim 1, wherein the one or more processors generate the first sub-prompt by segmenting the values into categorical values through single-value segmentation or multi-value segmentation, and performing segmental interpretation.

9. The medical prompt generation device of claim 1, wherein the one or more processors generate the first sub-prompt when the vector corresponding to the values or the element of the vector satisfies a predefined condition function.

10. The medical prompt generation device of claim 3, wherein the one or more processors generate the medical prompt of a sentence by inputting at least parts of the second sub-prompt and the third sub-prompt and the first sub-prompt into a language model.

11. A method of generating a medical prompt, performed by a medical prompt generation device comprising one or more processors, and one or more memories for storing instructions executed by the one or more processors, the method comprising generating a first sub-prompt based on values output by the artificial intelligence model.

12. The method of claim 11, further comprising inputting the first sub-prompt into a language model to generate a medical prompt.

13. The method of claim 11, further comprising generating a second sub-prompt based on the user-provided information; and generating a third sub-prompt based on a preset setting environment for setting a style of the medical prompt, wherein the generating of the medical prompt comprises generating the medical prompt based on the first sub-prompt and at least parts of the second sub-prompt and the third sub-prompt.

14. The method of claim 11, wherein the generating of the first sub-prompt comprises generating the first sub-prompt based on values in a vector format including, as an element, at least one of a type and a predicted probability of a disease expressed in a numerical form.

15. The method of claim 13, wherein the generating of the second sub-prompt comprises generating the second sub-prompt based on user-provided information including at least one of an age, a gender, a symptom, a medical history, an underlying disease, a social history, a differential diagnosis request, an emergency treatment request, a history taking request, a physical examination request, a medication request, and a treatment request.

16. The method of claim 13, wherein the generating of the third sub-prompt comprises generating the third sub-prompt based on a setting environment including at least one of a persona of the medical prompt, a context of the persona, and a setting of the persona.

17. The method of claim 13, wherein the values are output when the user-provided information is input to the artificial intelligence model.

18. The method of claim 11, wherein the generating of the first sub-prompt comprises generating the first sub-prompt by segmenting the values into categorical values through single-value segmentation or multi-value segmentation, and performing segmental interpretation.

19. The method of claim 11, wherein the generating of the first sub-prompt comprises generating the first sub-prompt when the vector corresponding to the values or the element of the vector satisfies a predefined condition function.

20. The method of claim 13, wherein the generating of the medical prompt comprises generating the medical prompt of a sentence by inputting at least parts of the second sub-prompt and the third sub-prompt and the first sub-prompt into a language model.
